# EUROPEAN PATENT SPECIFICATION

(11) **EP 2 684 559 B1**
(45) Date of publication and mention of the grant of the patent: **14.06.2017**
(21) Application number: 13164548.3
(22) Date of filing: 19.04.2013
(51) Int. Cl.: A61K 8/893, A61K 8/895, A61K 8/92, A61Q 1/02, A61Q 1/06, A61Q 1/08, A61Q 1/10, A61K 8/25, A61K 8/891, A61K 8/894

(54) **Oil-based makeup cosmetic preparation**
Kosmetische Makeup-Zubereitung auf Ölbasis
Préparation cosmétique de maquillage à base d'huile

(30) Priority: 25.04.2012 JP 2012099782
(43) Date of publication of application: 15.01.2014
(73) Proprietor: Shin-Etsu Chemical Co., Ltd., Tokyo (JP)
(72) Inventor: Inaba, Ryuichi, Tokyo (JP)
(74) Representative: Carpmaels & Ransford LLP

(56) References cited:
- EP-A1- 1 062 944
- EP-A1- 1 142 551
- EP-A1- 1 550 687
- EP-A1- 1 923 047
- JP-A- 2001 302 456
- US-A1- 2003 158 363

## Description

### Field of the invention

The present invention relates to an oil-based makeup cosmetic preparation.

### Description of the Related Art

Oil-based cosmetic preparations are, because of their excellent adhesiveness, water-tightness, and masking capability, widely used for lipsticks, eye-color, eye-liner, cheek-color, face-color, foundation and the like. Generally, the cosmetic preparation comprises pigments dispersed in a liquid, semi-solid, or solid base oil agent or in an oil base agent coagulated with a gelling-agent.

Conventional oil cosmetic preparations with a high content of the oil components, however, have the disadvantage of the user's discomfort due to their excessive sticky or oily touch. In order to overcome the disadvantage and to provide excellent feeling in use, is proposed an oil-based cosmetic preparation utilizing a gel composition comprising a polymerized silicone with a three-dimensional cross-linked molecular structure, and a low viscosity silicone oil (patent literature·1-3). Although the proposed cosmetic preparation is free of discomfort caused by the excessive sticky or oily touch, a new disadvantage arises due to a poor preservability, i.e., bleed-out of the oil components out of the cosmetic preparation, which contains dispersed pigments. An increased amount of the added cross-linked organopolysiloxane may prevent the bleed-out. The resulting oil-based makeup cosmetic preparation, however, has a problem of poor moistening and powdery touch.

Some oil-based makeup cosmetic preparations have been proposed that provide excellent feeling in use and have an excellent preservablity. The cosmetic preparations comprise an oil component containing a silicone oil, a cross-linked polyorganosiloxane, and an amorphous anhydrous silicic acid (patent literature 4, and 5). The oil-based makeup cosmetic preparations can spread smoothly when applied, achieve excellent feeling in use, and further acquire an excellent preservability. The performance of the cosmetic preparations, however, still remain unsatisfactory due to their poor dispersibility of the pigment-an essential component of a cosmetic preparation-and their poor durability of makeup, i.e., poor cosmetic wearability.

### Prior art documents

Patent literature 1 JP2561857B2
Patent literature 2 JP2561858B2
Patent literature 3 JP2582275B2
Patent literature 4 JP4523695B2
Patent literature 5 JP4647084B2

### Summary of the invention

An object of the present invention is to provide an oil-based makeup cosmetic preparation that can spread smoothly when applied, and achieve excellent feeling in use, excellent preservablitiy, pigment dispersibility and wearability.

As a result of intensive researches by the present inventor aiming at overcoming the above-mentioned technical problems, the following oil-based makeup cosmetic preparation was found to attain the object of the present invention.

The cosmetic preparation according to the invention comprises the following components in the following amounts relative to the entire mass of the cosmetic preparation:
(a) a partially cross-linked polyether-modified silicone, a partially cross-linked polyglycerin-modified silicone, or a combination thereof, in an amount of from 1 % by mass to 7.5% by mass;
(b) a low viscosity silicone oil with a viscosity of from 1 mm²/s and to 100 mm²/s at 25°C in an amount of from 3 % by mass to 75 % by mass;
(c) a cross-linked silicone powder in an amount of from 0.1 % by mass to 18 % by mass; and
(d) an amorphous anhydrous silicic acid with an average particle size of from 0.001 µm to 0.1 µm in an amount of from 0.1 % by mass to 5 % by mass.

### Effects of the invention

The oil-based makeup cosmetic preparation of the present invention can spread smoothly when applied and achieves excellent feeling in use, excellent preservablitiy, pigment dispersibility, wearability and removablility from the container.

### Detailed Description of the Preferred Embodiments

In the following is described the oil-based makeup cosmetic preparation of the present invention in detail. In the description, a viscosity referes to a value of kinetic viscosity measured with an Ostward viscometer at 25°C.

### <Component (a)>

The component (a) of the present invention is a partially cross-linked polyether-modified silicone, a partially cross-linked polyglycerin-modified silicone, or a combination thereof. Conventional partially cross-linked polyether-modified silicones or partially cross-linked polyglycerin-modified silicones may be employed in the present invention.

### •Partially cross-linked polyether-modified silicone

The partially cross-linked polyether-modified silicone is a three-dimensionally cross-linked product in which organopolysiloxane molecular chains are cross-linked by polyethers. Specific examples of the partially cross-linked polyether-modified silicones include those known under the following appellations: (Demithicone / (PEG-10/15)) cross-polymer; (PEG-15 / lauryl dimethicone) cross-polymer; (PEG-15 / lauryl polydimethylsiloxyethyl dimethicone) cross-polymer; and the like. They are swollen compositions comprising silicone oils or other oils, and commercially available under, for example, the following brand names: KSG-210; KSG-240; KSG-310; KSG-320; KSG-330; KSG-340; KSG-320Z; KSG-350Z ; or the like (all products of Shin-Etsu Chemical Co., Ltd.).

### •Partially cross-linked polyglycerin-modified silicone

The partially cross-linked polyglycerin-modified silicone is a three-dimensionally cross-linked product, wherein organopolysiloxane molecular chains are cross-linked by polyglycerine. Specific examples of the partially cross-linked polglycerin-modified silicones are those known under the following appellations: (dimethicone / polyglycerin-3) cross-polymer; (lauryl dimethicone / polyglycerin-3) cross-polymer; (polyglycerin-3/ lauryl polydimethylsiloxyethyl dimethicone) cross-polymer; and the like. They are swollen compositions comprising silicone oils or other oils, and commercially available under, for example, the following brand names: KSG-710; KSG-810; KSG-820; KSG-830; KSG-840; KSG-820Z; KSG-850Z; or the like (all products of Shin-Etsu Chemical Co., Ltd.).

The amount of the component (a) relative to the entire mass of the oil-based makeup cosmetic preparation is from 1 % by mass to 7.5 % by mass, and preferably from 2 % by mass to 7.5 % by mass. Too small amount will reduce the stability of the oil-based makeup cosmetic preparation and lead to inferior pigment dispersibility. Too large amount will deteriorate the removablity thereof from the container of the oil-based makeup cosmetic preparation and may deteriorate a light and smooth spread compromising an excellent feeling in use.

The component (a) may be used solely, or a combination of two or more thereof may be used. For example, when a partially cross-linked polyether-modified silicone and a partially cross-linked polyglycerin-modified silicones are combined, the higher content ratio of the polyether-modified silicone tends to make the resulting cosmetic preparation spread smoothly.
The higher content ratio of the polyglycern-modified silicone tends to develop a more moistening, soft touch of the cosmetic preparation. The content ratio of the cosmetic preparation can be determined depending on the required feeling in use.

The polar ether or glycerol structure included in the molecular structure of the partially cross-linked modified silicone of the component (a) remarkably enhances the dispersibility of the pigments contained in the cosmetic preparation. The pigments become harder to aggregate, leading to an enhanced stability of the cosmetic preparation and a better coloration of the cosmetic preparation applied on the skin. Moreover, since the hydrophilic structure of polyether or polyglycerol forms a cross-linked organopolysloxane main chain structure, the component (a) acts also as a low-HLB type surfactant. Consequently, the hydrophilic cross-linked parts capture water molecules, which makes the cosmetic preparation less likely to come off even after the cosmetic preparation is contaminated with or exposed to sweat or moisture on the skin, whereby imparting a better wearability to the cosmetic preparation.

The present preparation is excellent in gelling property, which imparts a favorable cosmetic feature of keeping the skin moistened to the preparation. The cosmetic preparation containing the present components is soft and free of a sticky touch, wherby achieving an excellent feeling in use. The soft feeling in use may be attributed, in one way, to the structure of the partially cross-linked polyether- or polyglycerin-modified silicone, and in another way, to the property comparable to a micro-sponge which traps oils. The trapping of nonvolatile oils imparts another feature of preventing color migration to the cosmetic preparation.

### <Component (b)>

The component (b) of the present invention is a silicone oil with a viscosity of from 1 mm²/s to 100 mm²/s at 25°C, and may be optionally selected, without particular limitation, among the silicone oils conventionally used in cosmetic preparations, that is, low viscosity organopolysiloxanes having linear, cyclic or branched molecular chains. Specific examples of the low viscosity silicone oil include: octamethyltrisiloxane and decamethyltetrasiloxane; (linear) diorganopolysiloxanes with the both molecular chain terminals blocked with triorganosiloxy groups, such as a dimethyl polysiloxane with the both molecular chain terminals blocked with trimethylsiloxy groups; cyclic diorganopolysiloxanes such as decamethylcyclopentasiloxane; and those known under the following appellations: trimethyl trimethicone; diphenyl dimethicone; and diphenylsiloxyphenyl trimethicone. For example, they are commercially available under the following brand names: KF-96A-1cs; KF-96L-1.5cs; KF-96L-2cs; KF-96A-5cs; KF-96A-6cs; KF-96A-10cs; KF-96A-20cs; KF-995; TMF-1.5; KF-53; KF-54; KF-56A; and the like. (all products of Shin-Etsu Chemical Co., Ltd.).

The amount of the component (b) relative to the entire mass of the oil-based makeup cosmetic preparation is from 3 % by mass to 75 % by mass, preferably from 3 % by mass to 65 % by mass, and more preferably from 5 % by mass to 65 % by mass. Too small amount may develop a powdery touch of the makeup cosmetic preparation, whereby leading to deteriorating a smooth spread when applied. Too large amount may develop an excessive oily touch, resulting in an uncomfortable feeling in use.

The component (b) may be used solely, or may be included in the component (a) as a swelling oil, or may be included by the addition of a commercial component (a)-containing swollen product that contains the component (b). The component (b) may be either volatile or non-volatile. The components (b) may be used solely, or a combination of two or more thereof may be used. When two or more kinds of components (b), i.e., volatile low viscosity silicone oils and nonvolatile low viscosity silicone oils, are combined, the higher content ratio of the volatile components tends to develop a powdery touch due to volatilization of the oil components when applied. The lower content ratio of the volatile components tends to develop a creamy touch due to the oil components remaining on the skin. The content ratio can be determined depending on the required feeling in use.

### <Component (c)>

The component (c) of the present invention is a cross-linked silicone powder, also known as a silicone rubber powder, comprising organopolysiloxanes with the molecular structure in which repeated chains of diorganosiloxane units are cross-linked. The conventional cross-linked silicone powders may be employed in the present invention and may be selected from the group consisting of silicone rubber powders and silicone resin-coated silicone rubber powders. The components (c) may be used solely, or a combination of two or more thereof may be used. Specific examples of the cross-linked silicone powders include those known under the following appellations: (dimethicone / vinyl dimethicone) cross-polymer; (vinyl dimethicone / methicone silsesquioxane) cross-polymer; (diphenyl dimethicone / vinyldiphenyl dimethicone / silsesquioxane) cross-polymer; polysilicone-22; polysilicone-1 cross-polymer; and the like. They are powders or swollen compositions containing silicone oils, and commercially available under, for example, the following brand names: KMP-400; KSP-100; KSP-101; KSP-102; KSP-105; KSP-300; KSP-411; KSP-441; KSG-016F; and the like (all products of Shin-Etsu Chemical Co., Ltd.). Incidentally, the partially cross-linked modified silicone of the component (a) is excluded from the component (c).

The amount of the component (c) relative to the entire mass of the oil-based makeup cosmetic preparation is from 0.1 % by mass to 18 % by mass, and preferably from 0.5 % by mass to 10 % by mass. Too small amount of the component (c) may deteriorate the preservability of the cosmetic preparation, whereas too large amount may result in inferior removability from the container of the preparation and in a powdery touch when applied.

The component (c) is preferably a cross-linked silicone powder capable of highly absorbing the oil components in the component (b). By including such cross-linked silicone powder having a high oil-absorbing property in the above-mentioned amount, the purpose of the invention can be sufficiently achieved. The components (c) may be solely used, or a combination of two or more thereof may be used. The amount of the oil components absorbed by the cross-linked silicone powder (c) can be measured according to the method provided by JIS K5101. When the measurement is carried out according to JIS K5101 using KF-96A-6cs (a dimethylpolysiloxane with a viscosity of 2 mm²/s at 25°C; a product of Shin-Etsu Chemical Co., Ltd.) which falls under the component (b), the amount of the dimethylpolysiloxane absorbed by 100 g of the silicone powder is particularly preferably 75g or more. Through incorporating in the above amount the component (c) capable of highly absorbing the oil components of the component (b), the cosmetic preparation of the present invention can attain all of the following properties: non-stickiness; smooth and soft touch; and an excellent preservability preventing an oil component-separation from the cosmetic preparation. Too low oil-absorbing property may result in a deteriorated stability of the oil-based makeup cosmetic preparation.

The component (c) preferably has a rubber hardness of less than 80. The rubber hardness may be measured with a type-A durometer as provided by JIS K6253. If the measured value, also known as "JIS A hardness" or "duro-A hardness", is too high, that means that the rubber powder is too rigid, a soft touch may not be imparted to the cosmetic preparation.

### (Component (d)〉

The component (d) of the present invention is an amorphous anhydrous silicic acid (amorphous silica fine powder) with an average particle size of from 0.001 µm to 0.1 µm. The term "an average particle size" refers to a mean volume diameter, which may be measured by the dynamic light scattering or by the Coulter counter method.

Examples of the amorphous anhydrous silicic acid used in the present invention include hydrophilic amorphous anhydrous silicic acid fine particles with a particle size within the above-mentioned range; amorphous anhydrous silicic acid fine particles with their surfaces subjected to a hydrophobilization-processing (i.e. hydrophilic or hydrophobic fumed silica), and the like. The hydrophilic particles are obtained thorough hydrolysis of normal silicon tetrachloride in oxyhydrogen flame. The hydrophobic fumed silica particles are obtained through processing the above hydrophilic particles so as to impart hydrophobicity to the particle surfaces. The amorphous anhydrous silicic acid may be hydrophilic or hydrophobic. Examples of the hydrophobilization-processing include trimethylsiloxidation with trimethylsilylchloride, hexamethyldisilazane or the like; methylation with dimethyldichlorosilane; a coating-baking treatment with a methylhydrogenpolysiloxane; and coating with a dimethylpolysiloxane, metal soap, and the like. Some of the amorphous anhydrous silicic acid fine particles are commercially available under the following brand names: Aerosil (registered trademark)-200; Aerosil-300; Aerosil-R972; Aerosil-R974; Aerosil-R202 (all products of Nippon Aerosil Co., Ltd.); and Tullanox 500 (product of Tulco Inc). The amount of the component (d) relative to the entire mass of the oil-based makeup cosmetic preparation of the present invention is in a range of from 0.1 % by mass to 5 % by mass, preferably from 0.5 % by mass to 3 % by mass. Inclusion of the amorphous anhydrous silicic acid in an amount within the above range enables the cosmetic preparation of the present invention to fully attain the object of the present invention. With the above amount of the component (d), the cosmetic preparation can achieve a favorable feeling in use without, particularly, a coarse touch and also achieve a favorable stability through preventing the oil component-separation from the cosmetic preparation.

### <Total amount of the components (a), (b), (c) and (d) in the entire cosmetic preparation>

The total amount of the components (a), (b), (c) and (d) of the present invention is within a range of from 4.2 % by mass to 100 % by mass. The amount within the range enables the resulting cosmetic preparation to fully attain the object of the present invention. An amount less than 4.2 % by mass may fail to achieve the effects of the present invention. As described later, the cosmetic preparation may contain various other components conventionally used for cosmetic prparations to the extent that they do not impair the effects of the present invention.

### <Component (e)>

The component (e) of the present invention is a film-forming agent and may be contained in the cosmetic preparation whenever necessary. There is no specific limitation on the silicone film-forming agent as long as it can form a water-resistant film on the skin after application of the cosmetic preparation to the skin and evaporation of the volatile oil components therein. Acrylic-silicone type graft copolymers and MQ resins are typical silicone film-forming agents, and other known silicone film-forming agents may be also employed.

The acrylic-silicone type graft copolymer is a graft copolymer having acrylic polymer chains grafted with dimethylpolysiloxane chains. One example is defined as ID: 10082 in the CTFA monograph. The graft copolymer is known for its main application as a film-forming agent under the appellation of (alkylacrylate / dimethicone) copolymer, and is commercially available under the following brand names: KP-545; KP-545L; KP-549; KP-550 (all products of Shin-Etsu Chemical Co., Ltd.); and the like. Those commercially available products contain the above copolymers dissolved in the following volatile solvents: isododecane; decamethylcyclopentasiloxane; methyl trimethicone; dimethicone (having a low molecular weight with a viscosity of no more than 2 mm²/s at 25 °C); and the like. Monomers used for producing the acrylic-silicone copolymer are preferably those having esterified carboxy groups in an acrylic acid because the resulting copolymers are reduced in acrylic odor.

The MQ resin is an organosilicone resin consisting of [R¹₃SiO_{1/2}] units (R¹ represents a hydrocarbon of 1 to 6 carbon atoms or phenyl group) and [SiO₂] units in a molar ratio of [R¹₃SiO_{1/2}]:[SiO₂] within a range of from 0.5:1 to 1.5:1. Examples of the hydrocarbon groups with 1 to 6 carbon atoms include alkyl groups such as methyl group, ethyl group, propyl group, butyl group, pentyl group, hexyl group. The MQ resin may be obtained through hydrolyzing a corresponding known silane compound and is known as "trimethylsiloxysilicic acid" and the like, and also known for its main application as a film-forming agent. The MQ resin is commercially available under the following brand names: KF-7312J; KF-9021; KF-7312K; KF-7312L; KF-7312T; X-21-5595; X-5616; (all products of Shin-Etsu Chemical Co., Ltd.) and the like. These commercially available products contain the resins dissolved in the following volatile solvents: isododecane; decamethylcyclopentasiloxane; methyl trimethicone; dimethicone (having a low molecular weight with a viscosity of no more than 2 mm²/s at 25 °C); and the like.

The component (e), a silicone film-forming agent, is optional, and contained in an amount of 50 % by mass or less relative to the entire mass of the cosmetic preparation. When the component is contained, the amount of the component (e) relative to the entire cosmetic preparation is preferably within the range of from 0.5 % by mass to 30 % by mass, more preferably from 1 % by mass to 15 % by mass. In the present invention, the above content of the component (e) can provide a favorable cosmetic durability, particularly an excellent transfer resistance.

### <Rheometer hardness>

The oil-based makeup cosmetic preparation of the present invention can be removed out of the container in an adequate portion directly with a finger or using an applicator such as a puff or a chip. At that time, the cosmetic preparation will enable the user to appreciate the perception of a light, airy scoop and a soft, smooth spread when applied on the skin. The elastic property (softness or hardness) of the cosmetic preparation of the present invention is preferably controlled so as to be within a desired range. The rheometer hardness may be measured with Rheometer RT-2002D·D (product of RHEOTEC Co. Ltd.) under the conditions of: a measuring terminal of 3 mmϕ; a penetration depth of 20 mm; a sample stage ascending speed of 2 cm/min; a temperature of 25 °C; and a range of 200. The hardness measured with the above rheometer under the above conditions is preferably less than 50. Among the essential components of the present invention, the components (a) and (c) greatly affect the rheometer hardness of the cosmetic preparation. The component (a) is preferably contained in an amount of no more than 7.5 % by mass relative to the entire mass of the cosmetic preparation, and the component (c) in an amount of no more than 18 % by mass. A content of the component (a) or (c) greater than the above value may fail to provide the perception of a light, airy scoop, and may make the user fail to perceive a soft, smooth spread when the cosmetic preparation is applied on the skin.

### <Other optional components>

The cosmetic preparation of the present invention may contain several other components used in conventional cosmetic preparations to the extent that the contained components do not impair the effects of the present invention. Such other components include (1) oils, (2) compounds having alcoholic hydroxyl groups, (3) powders, (4) surfactants, (5) compositions comprising a cross-linked organopolysiloxane and an oil that is liquid at room temperature, (6) silicone wax, and (7) other additives. Any of the above components may be employed solely or as a desired combination of two or more thereof.

### (1) Oily component

The oily component may be solid, semi-solid, or liquid. Natural vegetable or animal fats and oils, semi-synthetic fats and oils, hydrocarbon oils, higher alcohols, ester oils, silicone oils, or fluorine-containing oils, for example, may be employed.

### •Natural vegetable and animal fat and oil, and semi-synthetic fat and oil

Examples of natural vegetable and animal fats and oils, and semi-synthetic fats and oils include avocado oil, linseed oil, almond oil, ibota wax, perilla oil, olive oil, cacao butter, kapok wax, kaya oil, carnauba wax, cod liver oil, candelilla wax, refined cnadelilla wax, beef tallow, bovine hoof oil, bovine bone fat, hardened beef tallow, apricot kernel oil, spermaceti wax, hardened oils, wheat germ oil, sesame oil, rice germ oil, rice bran oil, sugarcane wax, sasanqua oil, safflower oil, shea butter, paulownia oil, cinnamon oil, jojoba wax, squalane, squalene, shellac wax, turtle oil, soybean oil, tea seed oil, camellia oil, evening primrose oil, corn oil, lard, rapeseed oil, Japanese tung oil, rice bran wax, germ oil, horse fat, persic oil, palm oil, palm kernel oil, castor oil, hardened castor oil, methyl esters of castor oil fatty acids, sunflower oil, grape seed oil, bayberry wax, jojoba oil, macadamia nut oil, yellow beeswax, mink oil, meadowfoam oil, cottonseed oil, cotton wax, Japan wax, sumac kernel oil, montan wax, coconut oil, hardened coconut oil, ocofatty acid triglycerides, mutton tallow, peanut oil, lanolin, liquid lanolin, reduced lanolin, lanolin alcohol, hardened lanolin, lanolin acetate, acetylated lanolin alcohol, isopropyl esters of lanolin fatty acids, POE lanolin alcohol ether, POE lanolin alcohol acetate, lanolin fatty acid polyethylene glycol, POE hydrogenated lanolin alcohol ether, and egg yolk oil, and the like.

### •Hydrocarbon oil

The hydrocarbon oils employed in the present invention include linear or branched hydrocarbon oils, and they may be volatile or non-volatile. Specific examples of the hydrocarbon oils include ozokerite, α-olefin oligomer, light isoparaffin, isododecane, isohexadecane, light liquid isoparaffin, squalane, synthetic squalane, vegetable squalane, squalene, ceresin, paraffin, paraffin wax, polyethylene wax, polyethylene•polypropylene wax, (ethylene / propylene / styrene) copolymer, (butylene / propylene / styrene) copolymer, liquid paraffin, liquid isoparaffin, pristane, polyisobutylene, hydrogenated polyisobutene, micro-crystalline wax, vaseline, and the like, and higher fatty acid such as lauric acid, myristic acid, palmitic acid, stearic acid, behenic acid, undecylenic acid, oleic acid, linoleic acid, linolenic acid, arachidonic acid, eicosapentaenoic acid (EPA), docosahexaenoic acid, isostearic acid, 12-hydroxystearic acid, and the like.

### •Higher alcohol

Examples of the higher alcohols usable in the present invention include alcohols having preferably six or more, more preferably 10 to 30 carbon atoms. More sepcific examples of the higher alcohols include lauryl alcohol, myristyl alcohol, palmityl alcohol, stearyl alcohol, behenyl alcohol, hexadecyl alcohol, oleyl alcohol, isostearyl alcohol, hexadodecanol, octyldodecanol, cetostearyl alcohol, 2-decyltetradecinol, cholesterol, phytosterol, polyoxyethylenecholesterol ether, monostearylglyceryl ether (batyl alcohol), monooleylglyceryl ether (selachyl alcohol), and the like.

### •Ester oil

Examples of the ester oils include esters such as diisobutyl adipate, 2-hexyldecyl adipate, di-2-heptylundecyl adipate, N-alkyl glyceryl monoiostearate, isocetyl isostearate, trimethylolpropane triisostearate, ethylene glyceryl di-2-ethylhexate, cetyl 2-ethylhexate, trimethylolpropane tri-2-ethylhexate, pentaerythritol tetra-2-ethylhexate, cetyl octante, octyldodecyl gum esters, oleyl oleate, octyldodecyl oleate, decyl oleate, neopentyl glyceryl dioctanate, neopentyl glyceryl dicaprate, triethyl citrate, 2-ethylhexyl succinate, amyl acetate, ethyl acetate, butyl acetate, isocetyl stearate, butyl stearate, diisopropyl sebacate, di-2-ethylhexyl sebacate. cetyl lactate, myristyl lactate, isononyl isononanoate, isotridecyl isononanoate, isopropyl palmitate, 2-ethylhexyl palmitate, 2-hexyldecyl palmitate, 2-heptylundecyl palmitate, cholesteryl 12-hydroxystearate, dipentaerythritol aliphatic acid esters, isopropyl myristate, octyldodecyl myristate, 2-hexyldecyl myristate, myristyl myristate, hexyldecyl dimethyloctanoate, ethyl laurate, hexyl laurate, 2-octyldodecyl N-lauroyl-L-glutamate, lauroyl sarcosine isopropyl ester, and diisostearyl malate; and glyceride oils such as glyceryl acetate, glycery triisooctanoate, glyceryl triisostearate, glyceryl triisopalmitate, glyceryl tribehenate, glyceryl monostearate, glyceryl di-2-heptylundecanoate, glyceryl trimyristate, diglyceryl myristyl isostearate, and the like.

### •Silicone oil

Examples of the silicone oils that are not included in the essential component (b) include dimetylpolysiloxane (excluding those included in the component (b)), caprylyl methicone phenyl trimethicone, methylphenylpolysiloxane, methylhexylpolysiloxane, methylhydrogenpolysiloxane, linear or branched organopolysiloxane with a viscosity within a wide range from low to high such as dimethylsiloxane-methylphenylsiloxane copolymer, amine-modified organopolysiloxane, pyrolidone-modified organopolysiloxane, pyrolidone-carboxylic-acid-modified organosiloxane, gum-like dimethylpolysiloxane with a high degree of polymerization, gum-like amine-modified organopolysiloxane, a solution of silicone rubber such as gum-like dimethylsiloxane-methylphenylsiloxane copolymer in a cyclic organopolysiloxane, a solution of a silicone gum or rubber in a cyclic organopolysiloxane, trimethylsiloxysilicate, a solution of trimethylsiloxysilicate in a cyclic siloxane, higher alcohol-modified silicone such as stearoxyl silicone, higher aliphatic acid-modified silicones, alkyl-modified silicones, long chain alkyl-modified silicones, amine-modified silicones, fluorine-modified silicones, a solution of silicone resin, and the like.

### •Fluorine-based oil

Examples of the fluorine-based oils include perfluoropolyether, perfluorodecalin, perfluorooctane, and the like.

The content of the oils in the entire cosmetic preparation may be properly determined depending on the formulation type of the cosmetic preparation within a range of from 1 to 95.8 % by mass.

### (2) Compound with alcoholic hydroxyl groups

Examples of the compounds with alcoholic hydroxyl groups include: lower alcohols with 2 to 5 carbon atoms, such as ethanol and isopropanol; sugar alcohols such as sorbitol and maltose; and the like. Other examples include: sterols such as cholesterol, sitosterol, phytosterol and lanosterol; polyhydric alcohols such as butylene glycol, propylene glycol, dibutylene glycol and penthylene glycol; and the like.

The amount of the compound having alcoholic hydroxyl groups in the entire cosmetic preparation may be properly determined within a range of from 0.1 to 10 % by mass.

### (3) Powder

Examples of the powders that may be employed in the present invention include inorganic particles, organic particles, and inorganic-organic composite powders excluding those included in the component (c) or component (d) of the present invention. Specific examples are as follows.

### •Inorganic particles not included in the component (d)

Specific examples of the inorganic particles include fine particles of titanium oxide, titanated mica, zirconium oxide, zinc oxide, cerium oxide, magnesium oxide, barium sulfate, calcium sulfate, magnesium sulfate, calcium carbonate, magnesium carbonate, talc, cloven talc, mica, kaolinite, sericite, white mica, synthetic mica, phlogopite, lepiodite, biotite, lithia mica, silicic acid, silicon dioxide, fumed silica, hydrous silicon dioxide, aluminium silicate, magnesium silicate, aluminium magnesium silicate, calcium silicate, barium silicate, strontium silicate, metal tungstate, hydroxyl apatite, vermiculite, higilite, bentonite, montmorillonite, hectorite, zeolite, ceramics, dicalcium phosphate, alumina, aluminium hydroxide, boron nitride, glass, or the like.

Other examples of the inorganic fine particles include pigment-type inorganic fine particles, more specifically, inorganic red pigment such as iron oxide, iron hydroxide, and iron titanate; inorganic blown pigments such as γ-iron oxide; inorganic yellow pigments such as yellow iron oxide, and ocher; inorganic black pigments such as black iron oxide, and carbon black; inorganic purple pigments such as manganese violet and cobalt violet; inorganic green pigments such as chromium hydroxide, chromium oxide, cobalt oxide and cobalt titanate; inorganic blue pigments such as Berlin blue and ultramarine blue; colored pigments such as laked tar coloring matter and laked natural color; and pearl pigments such as titanium oxide-coated mica, bismuth oxychloride, titanium oxide-coated bismuth oxhychloride, titanium oxide-coated talc, fish scale essence, and titanium oxide-coated colored mica.

Other examples also include metal fine particles of aluminium, copper, stainless-steel, or silver.

### •Organic particles not included in the component (c)

Examples of the organic particles include powders made from polyamides, polyacrylic acids, polyacrylates, polyesters, polyethylenes, polypropylenes, polystyrenes, styrene-acrylic acid copolymers, divinylbenzene-styrene copolymers, polyurethanes, polyvinyl resins, urea-resins, melamine resins, benzoguanamine, polymethylbenzoguanamines, tetrafluoroethylene, polymethylmetacrylates, cellulose, silk, nylon, phenol resins, epoxy resins, polycarbonates, or the like.

Other examples of the organic particles include metallic soaps, more specifically, a powder made from zinc stearate, aluminium stearate, calcium stearate, magnesium stearate, zinc myristate, magnesium myristate, zinc cetyl phosphate, calcium cetyl phosphate, zinc sodium cetyl phosphate, or the like.

Other examples of the organic particles include organic pigment, more specifically: a tar coloring matter such as Red No.3, Red No.104, Red No.106, Red No.201, Red No.202, Red No.204, Red No.205, Red No.220, Red No.226, Red No.227, Red No.228, Red No.230, Red No.401, Red No.505, Yellow No.4, Yellow No.5, Yellow No.202, Yellow No.203, Yellow No.204, Yellow No.401, Blue No.1, Blue No.2, Blue No.201, Blue No.404, Green No.3, Green No.201, Green No.204, Green No.205, Orange No.201, Orange No.203, Orange No.204, Orange No.206, Orange No.207, and the like; and a natural pigment such as carminic acid, laccaic acid, carthamine, brazilin, crocin, and the like.

The other organic particles include silicone resin particles except for those included in the component (c), e.g., polyorganosilsesquioxane resin particle having a three dimensional network molecular structure, and their examples include polymethylsilsesquioxane, more specifically KMP-590, and KMP-591 (products of Shine-Etsu Chemical Co., Ltd.).

### •Inorganic-organic composite powder

Examples of the inorganic-organic composite powders include those composite powders such as an inorganic powder that is generally used for cosmetics and has its surfaces coated with an organic powder in a known method.

The above mentioned powders of the present invention not included in the component (c) may comprise particles having their surfaces treated with a hydrogen silicide such as caprylsilane (AES-3083; productr of Shin-Etsu Chemical Co., Ltd.) or a silylation agent; a silicone oil such as dimetylsilicone (KF-96AK group; product of Shin-Etsu Chemical Co., Ltd.) methylhydrogenpolysiloxanes (KF-99P, KF-9901; products of Shin-Etsu Chemical Co., Ltd.), and branched silicone based silicone-processing agent (KF-9908, KF-9909; products of Shin-Etsu Chemical Co., Ltd.); waxes; paraffins; organofluorine compounds such as perfluoroalkylphospate; surfactants; amino acids such as N-acylblutamine; and metal soaps such as aluminium stearate and magnesium myristate.

There is no limitation on the content of the powders, which may be as high as about 100 % by mass relative to the total mass of the cosmetic preparation.

### (4) Surfactant

The surfactant may be nonionic, anionic, or cationic. There is no specific limitation on the surfactant and any of those used for conventional cosmetic preparations may be used. Examples of the preferable surfactants include linear or branched polyoxyethylene-modified organopolysiloxanes, linear or branched polylxyiethylene-polyoxypropylene-modified organopolysiloxanes, linear or branched polyoxyethylene- and alkyl-co-modified organopolysiloxanes, linear or branched polyoxyethylene-, polypropylene- and alkyl-co-modified organopolysiloxanes, linear or branched polyglycerin-modified organopolysiloxanes, and linear or branched polyglycerin- and alkyl- co-modified organopolysiloxanes. The surfactant preferably has hydrophilic polyoxyethylene groups, polyoxyethylenepolyoxypropylene groups or polyglycerin groups in a content of 10 to 70 % by mass per one molecule. Specific examples include KF-6011, KF-6043, KF-6028, KF-6038, KF-6100, KF-6104, KF-6105 (all products of Shin-Etsu Chemical Co., Ltd.), and the like.

The content of the surfactant relative to the total mass of the entire cosmetic preparation is preferably within a range of from 0.1 % by mass to 20 % by mass, more preferably from 0.2 % by mass to 10 % by mass. The HLB of the surfactant may not be limited to a specific values, but is preferably within a range of from 2 to 14.5.

### (5) Composition comprising cross-linked organopolysiloxane and oil that is liquid at room temperature

In the composition comprising a cross-linked organopolysiloxane and an oil that is liquid at a room temperature, the cross-linked organopolysiloxane preferably swells upon taking up the liquid oil in the same amount of the weight of the organopolysiloxane or more. The usable liquid oils include the silicone oil, hydrocarbon oils, ester oils, natural vegetable and animal oils, semi-synthetic oils and fluorine-containing oils included in the component (1) mentioned above. Examples of the oils include a low viscosity silicone oil with a viscosity within a range of from 0.65mm²/s to 100 mm²/s at 25°C; hydrocarbon oils such as liquid paraffin, squalane, isododecane and isohexadecane; glyceride oil such as trioctanoin; ester oil such as isotridecyl isononanoate, N-acylglutamic acid ester, and lauroylsarcosine acid ester; and natural vegetable and animal oils such as macadamia nut oil. Unlike the component (a) of the present invention, the molecular structure of the component (5) has neither a polyether nor polyglycerol structure. Specific examples of the component (5) include KSG group (brand name of the product of Shin-Etsu Chemical Co., Ltd.), particularly KSG-15, KSG-16, KSG-19, KSG-41, KSG-42, KSG-43, KSG-44, KSG-042Z, KSG-045Z.

A content of the composition (5) comprising the cross-linked organopolysiloxane and the liquid oil relative to the total mass of the entire cosmetic preparation is preferably within a range of from 0.1 % by mass to 80 % by mass, more preferably from 1 % by mass to 50 % by mass.

### (6) Silicone wax

The silicone wax of the component (6) is preferably an acrylic silicone resin consisting of an acrylic silicone graft or block copolymer. Also may be used an acrylic silicone resin that includes in its molecule at least one moiety selected from a pyrolidone moiety, a long-chain alkyl moiety, a polyoxyalkylene moiety, a fluoroalkyl moiety, and an anion moiety such as a carboxylic acid. Specific examples of the component (6) include acrylic silicone graft copolymers, e.g., KP-561P and KP-562P (products of Shin-Etsu Chemical Co., Ltd.). The silicone wax is preferably a polylactone-modified polysiloxane, in which the bonded polylactone is obtained through ring-opening polymerization of a lactone compound having five or more ring member. Moreover, the silicone wax may be a silicone-modified olefin wax that is obtainable through an addition reaction between an olefin wax and an organohydrogenpolysiloxane, wherein the olefin has unsaturated groups and comprises an α-olefin and a diene compound, and the organohydrogenpolysiloxane has one or more Si-H bond per one molecule. Preferable α-olefin in the olefin wax is an olefin having 2 to 12 carbon atoms such as ethylene, propylene, 1-butene, 1-hexene and 4-methyl-1-pentene, and preferable diene compounds include butadiene, isoprene, 1,4-hexadiene, vinylnorbornene, ethylidenenorbornene and dicyclopentadiene. The Si-H bond-containing organohydrogenpolysiloxane may have a linear siloxane structure or a branched siloxane structure.

When the silicone wax is used, a content of the wax relative to the total mass of the entire cosmetic preparation is preferably within a range of from 0.1 % by mass to 30 % by mass, more preferably from 1 % by mass to 10 % by mass.

### (7) Other additives

The other additives included in the component (7) refer to an oil-soluble gelling agent, antiperspirant, UV absorber, UV absorbing/scattering agent moisturizer, antibacterial antiseptic, perfume, salts, antioxidizing agent, pH-controller, chelating agent, refrigerant, anti-inflammatory agent, beauty skin component, i.e., skin lightner, cell activator, rough-skin treatment agent, blood circulation promoter, skin astringent, and antiseborrheic, vitamins, amino acids nucleic acid, hormones, clathrate compound, and the like.

### •Oil-soluble gelling agent

The oil-soluble gelling agent of the present invention refers to a metal soap such as aluminium stearate, magnesium stearate and zinc myristate; an amino acid derivative such as N-lauroyl-L-glutamic acid and α, γ-di-n-butylamine; a dextrine fatty acid ester such as dextrine palmitiate ester, dextrine stearate ester and dextrine 2-ethtylhexanoate-palmitate ester; a saccharose fatty acid ester such as saccharose palmitate ester and saccharose stearate ester; a fructo-oligosaccharide fatty acid ester such as fructo-oligosaccharide stearate ester and fructo-oligosaccharide 2-ethylhexanoate ester; a sorbitol derivative with benzylidene such as monobenzylidene sorbitol and dibenzylidene sorbitol; and an organic modified clay ore such as dimethylbenzyldodecylammonium montmorillonite clay and dimethyloctadecylammonium montmorillonite clay.

### •Antiperspirant

The antiperspirant in the present invention refers to aluminium chlorohydrate, aluminium chloride, aluminium sesquichlorohydrate, zirconylhydroxychloride, aluminium zirconium hydroxychloride, aluminium zirconium glycin complex, and the like.

### •UV absorber

The UV absorber in the present invention refers to a benzoic acid-based UV absorber such as p-aminobenzoic acid; an anthranilic acid-based UV absorber such as methyl anthranilate; a salicylic acid-based UV absorber such as methyl salicylate, octyl salicylate and trimethylcyclohexyl salicylate; a cinnamic acid-based UV absorber such as octyl-p-methoxycinnamate; a benzophenone-based UV absorber such as 2,4-dihydroxybenzophenone; an urocanic acid-based UV absorber such as ethyl urocanate; a dibenzoylmethane-based UV absorber such as 4-t-butyl-4'-methoxydibenzoylmethane; phenylbenzimidazolesufonic acid; triazine derivative; and the like.

### •UV absorbing/scattering agent

The UV absorbing/scattering agent in the present invention refers to particles which can absorb/scatter ultraviolet rays, such as titanium oxide fine particles, iron-containing titanium oxide fine particles, zinc oxide fine particles, cerium oxide fine particles, and their composites. The above particles capable of absorbing/scattering particles may be dispersed in an oil before use to provide a usable dispersion.

### •Moisturizer

The moisturizer in the present invention refers to glycerin, sorbitol, propylene glycol, dipropylene glycol, 1,3-butylene glycol, pentylene glycol, glucose, xylitol, maltitol, polyethylene glycol, hyaluronic acid, chondroitin sulfuric acid, pyrrolidone carboxylic acid, polyoxyethylene methylglucoside, polyoxypropylene methylglucoside, egg-yolk lecithin, soy lecithin, phosphatidylcholine, phosophatidylethanol amine, phosphatidylserine, phospahtidyl glycerol, phosphatidyl inositol, and sphingophospholipid, and the like.

### •Antibacterial antiseptic

The antibacterial antiseptics in the present invention refer to an alkyl p-oxybenzoate, benzoic acid, sodium benzoate, sorbic acid, potassium sorbate, phenoxyethanol, and the like. The antibacterials of the present invention refer to benzoic acid, salicylic acid, phenol, sorbic acid, an alkyl p-oxybenzoate, p-chloro-m-cresol, hexachlorophene, benzalkonium chloride, chlorhexidine chloride, trichlorocarbanilide, photosensitizer, phenoxyethanol, and the like.

### •Perfume

The perfumes in the present invention include natural and synthetic perfumes. The natural perfumes refer to vegetable perfumes extracted from flowers, leaves, stems, fruit skin and the like; and animal perfumes such as musk and civet. The synthetic perfumes refer to hydrocarbons such as monoterpene; alcohols such as aliphatic alcohols and aromatic alcohols; aldehydes such as terpene aldehyde and aromatic aldehyde; ketones such as alicyclic ketones; esters such as terpene-based ester; lactones; phenols; oxides; nitrogen-containing compounds; acetals; and the like.

### •Salts

The salts in the present invention refer to inorganic salts, organic salts, amine salts, and amino-acid salts. Examples of the inorganic salts include metallic salts of inorganic acids such as a hydrochloride, sulfate, carbonate, nitrate, and the like, each containing sodium, potassium, magnesium, calcium, alminium, zirconium, zinc or the like. Examples of the organic salts include salts of an organic acid such as acetic acid, dehydroacetic acid, citric acid, malic acid, succinic acid, ascorbic acid, stearic acid, and the like. Example of the amine salts and amino-acid salts include salts of amines such as triethanolamine, and salts of amino acids such as glutamic acid. The other alternatives include salts of hyaluronic acid, chondroitin sulfuric aicd and the like; and aluminium zirconium glycine complexes, and the like. Another usable salts may be acid-base neutralized salts used for preparing cosmetics.

### •Antioxidizing agent

The antioxidizing agent in the present invention refers to tocopherol, p-t-butyl phenol, butyl hydroxy anisole, dibutyl hydoxy tolunene, phytic acid, and the like.

### •pH-adjuster

The pH-adjuster in the present invention refers to lactic acid, citric acid, glycolic acid succinic acid, tartaric acid, d1-malic acid, potassium carbonate, sodium bicarbonate, ammonium bicarbonate, and the like.

### •Chelating agent

The chelating agent in the present invention refers to alanine, sodium edetate, sodium polyphosphate, sodium metaphosphate, phosphoric acid, and the like.

### •Refrigerant

The refrigerant in the present invention refers to L-menthol, camphor, and the like.

### •Anti-inflammatory agent

The anti-inflammatory agent in the present invention refers to allantoin, glycyrrhizinic acid and its salts, glycyrrhetinic acid, stearyl glycyrrhetinate, tranexamic acid, azulene, and the like.

### •Beauty skin component

The beauty skin components in the present invention refer to skin whiteners such as placenta extract, arbutin, glutathione and saxifrage extracts; cell activators such as royal jelly, phtosensitizers, cholesterol derivatives and calfblood extracts; rough-skin treatment agents; blood circulation promoter such as nonyl acid vanillyl amide, benzyl nicotinate β-butoxyethyl nicotinate, capsaicin, zingerone, cantharis tincture, ichthammol, caffeine, tannic acid, α-borneol, tocopherol nicotinate, inositol hexanicotinate, cyclandelate, cinnarizine, tolazoline, acetylcholine, verapamil, cepharanthin and γ-orizanol; skin astringents such as zinc oxide and tannic acid; antiseborrheics such as sulfur and thianthol; and the like.

The vitamins in the present invention refer to vitamin As such as vaitamin A oil, retinol, retinol acetate and retinol palmitate; vitamin B₂s such as riboflavin, riboflavin acetate and flavin adenine nucleotide; vitamin B₆s such as pyridoxine hydrochloride, pyridoxine dioctanoate and pyridoxine dipalmitate; vitamin Bs such as vitamin B₁₂ and its derivatives, and vitamin B₁₅ and its derivatives; vitamin Cs such as L-ascorbic acid, L-ascorbate dipalmitate ester, sodium L-ascorbate-2-sulfate and dipotassium L-ascorbate phosphate diester; vitamin Ds such as erogocalciferol and chlecalciferol; vitamin Es such as α-tocopherol, β-tocopherol, γ-tocopherol, dl-α-tocopherol acetate, dl-α-tocopherol nicotinate and dl-α-tocopherol succinate; nicotinic acids such as nicotinic acid, benzyl nicotinate and nicotinic-acid amide; vitamin H; vitamin P; pantothenic acid salts or derivatives such as potassium pantothenate, D-pantothenyl alcohol, pantothenylethyl ether, and acetylpantothenylethyl ether; biotins; and the like.

### •Amino acids

The amino acids in the present invention refer to glycine, valine, leucine, isoleucine, serine, threonine, phenylalanine, arginine, lysine, asparaginic acid, glutamic acid, cystine, cysteine, methionine, and tryptophane.

### •Nucleic acid

The nucleic acids in the present invention refer to deoxyribo nucleic acid and the like.

### •Hormone

The hormones in the present invention refer to estradiol and ethenylestradiol. •Clathrate compound

The clathrate compounds in the present invention refer to cyclodextrin and the like.

The cosmetic preparation of the present invention is applicable to various oil-based makeup cosmetics. Examples of the oil-based makeup cosmetics include makeup base, concealer, foundation, cheek-color, eye-color, mascara, eye-liner, eyebrow, lip stick, and the like. The cosmetic preparation can take various forms such as cream, cake, paste, gel, mousse, and soufflé, which provide a wide range of choice.

### Example

The following examples and comparative examples describe the details of the present invention, which should not be construed to limit the present invention. In the examples, the values of viscosities are measured at 25°C, and the "%" representing a concentration or content indicates "% by mass".

### <Example 1 and Comparative examples 1 to 4> Oil-based mousse foundation

Oil-based mousse foundations were prepared according to the formulations shown in Table 1.

**[Table 1]**

| Component | | | % by mass | | | | |
|---|---|---|---|---|---|---|---|
| | | | Example | Comparative example | | | |
| | | | 1 | 1 | 2 | 3 | 4 |
| 1 | partially cross-linked polyether-modified silicone swollen composition¹⁾ | (a) | 3.60 | 8.00 | 3.60 | 3.60 | 0.00 |
| | | (b) | 14.40 | 32.00 | 14.40 | 14.40 | 0.00 |
| 2 | dimethylpolysiloxane²⁾ | (b) | 12.00 | 12.00 | 12.00 | 12.00 | 1.20 |
| 3 | neopentyl glycol dioctanoate | | 5.00 | 5.00 | 5.00 | 5.00 | 5.00 |
| 4 | organosilicone resin solution³⁾ | (b) | 2.50 | 2.50 | 2.50 | 2.50 | 2.50 |
| | | (e) | 2.50 | 2.50 | 2.50 | 2.50 | 2.50 |
| 5 | decamethylcyclopentasiloxa ne⁴⁾ | (b) | 25.08 | 3.08 | 25.08 | 25.58 | 39.48 |
| 6 | amorphous anhydrous silicic acid ⁵⁾ | (d) | 0.50 | 0.50 | 0.50 | 0.00 | 0.50 |
| 7 | hybrid silicone composite powder⁶⁾ | (c) | 6.00 | 6.00 | 0.00 | 6.00 | 6.00 |
| 8 | polymethylsilsesquioxane⁷⁾ | | 3.00 | 3.00 | 3.00 | 3.00 | 3.00 |
| 9 | polymethyl methacylate spherical particle powder | | 7.00 | 7.00 | 13.00 | 7.00 | 7.00 |
| 10 | alkylsilicone-branched silicone⁸⁾-treated iron oxide | | 1.22 | 1.22 | 1.22 | 1.22 | 1.22 |
| 11 | alkylsilicone-branched silicone⁸⁾-treated titanium oxide | | 5.00 | 5.00 | 5.00 | 5.00 | 5.00 |
| 12 | alminium stearate-treated particulate titanium oxide | | 5.00 | 5.00 | 5.00 | 5.00 | 5.00 |
| 13 | tocopherol | | 0.20 | 0.20 | 0.20 | 0.20 | 0.20 |
| 14 | alkylsilicone-branched silicone⁸⁾-treated talc | | 3.50 | 3.50 | 3.50 | 3.50 | 3.50 |
| 15 | alkylsilicone-branched silicone⁸⁾-treated sericite | | 3.50 | 3.50 | 3.50 | 3.50 | 3.50 |
| 16 | partially cross-linked unmodified silicone swollen composition⁹⁾ | | 0.00 | 0.00 | 0.00 | 0.00 | 3.6 |
| | | (b) | | | | | 10.8 |
| Total | | | 100.00 | 100.00 | 100.00 | 100.00 | 100.00 |
| total of component (a) | | | 3.60 | 8.00 | 3.60 | 3.60 | 0.00 |
| total of component (b) | | | 53.98 | 49.58 | 53.98 | 54.48 | 53.98 |
| total of component (c) | | | 6.00 | 6.00 | 0.00 | 6.00 | 6.00 |
| total of component (d) | | | 0.50 | 0.50 | 0.50 | 0.00 | 0.50 |
| total of component (e) | | | 2.50 | 2.50 | 2.50 | 2.50 | 2.50 |
| rheometer hardness | | | 10 | 61 | 0 | 13 | 12 |

| | | | | | | | |
|---|---|---|---|---|---|---|---|
| 1) KSG-240 (product of Shin-Etsu Chemical Co., Ltd.); a composition containing a partially cross-linked polyether-modified silicone swollen with decamethylcyclopentasiloxane; partially cross-linked polyether-modified silicone : decamethylcyclopentasiloxane = 20:80 (wt/wt) 2) KF-96A-6cs (product of Shin-Etsu Chemical Co., Ltd.); dimethylpolysiloxane with a viscosity of 6 mm²/s at 25° C 3) KF-7312J (product of Shin-Etsu Chemical Co., Ltd.); a solution of an organosilicone resin in decamethylcyclopentasiloxane; trimethylsiloxysilicic acid : decamethylcyclopentasiloxane = 50:50 (wt/wt) 4) KF-995 (product of Shin-Etsu Chemical Co., Ltd.); decamethylcyclopentasiloxane 5) AEROSIL R972 (product of Nippon Aerosil Co., Ltd.); amorphous anhydrous silicic acid 6) KSP-411 (product of Shin-Etsu Chemical Co., Ltd.); hybrid silicone composite powder 7) KMP-590 (product of Shin-Etsu Chemical Co., Ltd.); polymethylsilsesquioxane 8) KF-9909 (product of Shin-Etsu Chemical Co., Ltd.); alkylsilicone-branched silicone 9) KSG-16 (product of Shin-Etsu Chemical Co., Ltd.); a composition containing a partially cross-linked unmodified silicone swollen with dimethylpolysiloxane; partially cross-linked unmodified silicone: dimethylcyclopolysiloxane = 25:75 (wt/wt) | | | | | | | |

### -Preparation method-

Foundations were prepared through the method comprising the following two steps:
step A: evenly dispersing the components 1 to 4, 6, 10 to 12 and 16 in Table 1 by means of a roll mill; and
step B: evenly dispersing the components 5, 7 to 9 and 13 to 15 in Table 1 in the mixture obtained in the step A.

### -Measurement of rheometer hardness-

The rheometer hardness of each of the foundaions obtained in Example 1 and Comparative examples 1 to 4 were measured with Rheometer RT-2002D· D (product of RHEOTECH Co., Ltd.) under the conditions of:
a measuring terminal of 3 mmϕ;
a penetration depth of 20 mm;
a sample stage ascending speed of 2 cm/min;
a temperature of 25°C; and
a range of 200.

The foundation of Example 1 exhibited a rheometer hardness of 10 and exhibited an excellent removablity, a soft and light spread, and was free of excessive oiliness or powdery touch, providing an excellent feeling in use. The foundation also exhibited an excellent water-resistance, water-repellency, and perspiration resistance, as well as a favorable wearability, whereby making a cosmetic not easily come off. The foundation of Example 1 was stored at room temperature, 5° C and 50° C. One month later, the evaluation on oil bleed-out and usability of the stored preparations exhibited no changes in the properties caused by aging. An excellent stability of the foundation was proved.

### -Property evaluation-

The properties of the foundations of Example 1 and Comparative examples 1 to 4 were evaluated based on the criteria shown in Table 2. The evaluated properties were removability of the preparation, spread on application (spreadability), softness (feeling in use), attachement (adhesiveness and evenness), miscibility of powder (dispersibility), makeup wearability (cosmetic effect durability) and preservability. The results of the evaluation by ten panelists were averaged. The average values were rated based on the following evaluation criteria. The results are shown in Table 3.

**[Table 2]**

| Item | Score | | | | |
|---|---|---|---|---|---|
| | 5 | 4 | 3 | 2 | 1 |
| removability | good | slightly good | fair | slightly poor | poor |
| spread | good | slightly good | fair | slightly poor | poor |
| softness | good | sightly good | fair | slightly poor | poor |
| attachment | good | slightly good | fair | slightly poor | poor |
| miscibility | good | slightly good | fair | slightly poor | poor |
| wearability | good | slightly good | fair | slightly poor | poor |
| preservability | good | slightly good | fair | slightly poor | poor |

### -Evaluation criteria-

A : The average value is no less than 4.5.
B : The average value is no less than 3.5 but less than 4.5.
C : The average value is no less than 2.5 but less than 3.5.
D : The average value is no less than 1.5 but less than 2.5.
E : The average value is less than 1.5.

**[Table 3]**

| Item | Example 1 | Comparative example 1 | Comparative example 2 | Comparative example 3 | Comparative example 4 |
|---|---|---|---|---|---|
| removability | A | E | A | A | A |
| spread | A | E | A | D | A |
| softness | A | C | C | A | A |
| attachment | A | A | D | A | B |
| miscibility | A | B | A | A | C |
| wearability | A | A | D | C | C |
| preservability | A | B | E | D | C |

Table 3 clearly shows that the foundation of Example 1 has a better removability, spread on application (spreadability), softness (feeling in use), attachment (adhesiveness and evenness), miscibility of powder (dispersibility), makeup wearability (cosmetic effect durability), and preservablitiry than any foundation of Comparatives 1 to 4. Superiority of the foundation of Example 1 was proved.

### <Example 2> Cheek-color

A cheek-color was preapered according to the formulations shown in Table 4.

**[Table 4]**

| Component | | | % by mass |
|---|---|---|---|
| 1 | partially cross-linked polyether-modified silicone swollen composition¹⁾ | (a) | 4.25 |
| | | (b) | 12.75 |
| 2 | decamethylcyclopentasiloxane | (b) | 48.8 |
| 3 | neopentyl glycol dioctanoate | | 9.0 |
| 4 | stearoyl inulin | | 2.0 |
| 5 | hybrid silicone composite powder²⁾ | (c) | 5.0 |
| 6 | amorphous anhydrous silicic acid³⁾ | (d) | 1.0 |
| 7 | red No.202 | | adequate |
| 8 | alkylsilicone-branched silicone⁴⁾-treated iron oxide | | adequate |
| 9 | alkylsilicone-branched silicone⁴⁾-treated titanium oxide | | adequate |
| 10 | tocopherol | | 0.2 |
| 11 | alkylsilicone-branched silicone⁴⁾-treated titanated mica | | 6.0 |
| 12 | alkylsilicone-branched silicone⁴⁾-treated mica | | 10.0 |
| Total | | | 100.0 |
| total of component (a) | | | 4.25 |
| total of component (b) | | | 61.55 |
| total of component (c) | | | 5.0 |
| total of component (d) | | | 1.0 |

| | | | |
|---|---|---|---|
| 1) KSG-210 (product of Shin-Etsu Chemical Co., Ltd.); a composition containing a partially cross-linked polyether-modified silicone swollen with dimethylpolysiloxane; partially cross-linked polyether-modified silicone : dimethylpolysiloxane = 20:75 (wt/wt) 2) KSP-100 (product of Shin-Etsu Chemical Co., Ltd.); hybrid silicone composite powder 3) AEROSIL R200 (product of Nippon Aerosil Co., Ltd.); amorphous anhydrous silicic acid 4) KF-9909 (product of Shin-Etsu Chemical Co., Ltd.); alkylsilicone-branched silicone | | | |

### -Preparation method-

The cheek-color was prepared through the method comprising the following three steps:
step A: heat-mixing the components 1 to 4 and 6 in Table 4 at 80°C, followed by evenly dispersing the mixed components by a roll mill;
step B: evenly dispersing the components 5 and 7 to 12 in Table 4 into the mixture obtained in the step A at 80°C; and
step C: cooling down the mixture obtained in the step B to room temperature.

The cheek-color obtained in Example 2 in the form of a soufflé exhibited an excellent removablity and a light spread, and was free of excessive oiliness and powdery touch, providing excellent feeling in use. The cheek-color also had an excellent water-resistance, water-repellency and perspiration resistance, as well as a favorable wearability, which makes a cosmetic not easily come off. Further, the cheek-color exhibited no change in the properties caused by temperature variation or aging. An excellent preservability of the cheek-color was proved.

### <Example 3> Foundation

A foundation was prepared according to the formulations shown in Table 5

**[Table 5]**

| Component | | | % by mass |
|---|---|---|---|
| 1 | decamethylcyclopentasiloxane | (b) | 40.1 |
| 2 | (alkyl acrylate / dimethicone) copolymer solution¹⁾ | (b) | 3.5 |
| | | (e) | 1.5 |
| 3 | partially cross-linked polyglycerin-modified silicone swollen composition²⁾ | (a) | 4.0 |
| | | (b) | 12.0 |
| 4 | hybrid silicone composite powder³⁾ | (c) | 8.0 |
| 5 | squalane | | 1.0 |
| 6 | jojoba oil | | 1.0 |
| 7 | diphenylsiloxyphenyl trimethicone⁴⁾ | (b) | 1.0 |
| 8 | polymethyl metacrylate spherical particle powder | | 10.0 |
| 9 | amorphous anhydrous silicic acid⁵⁾ | (d) | 0.5 |
| 10 | alkylsilicone-branched silicone⁶⁾-treated iron oxide | | 1.2 |
| 11 | alkylsilicone-branched silicone⁶⁾-treated titanium oxide | | 5.0 |
| 12 | aluminium stearate-treated particulate titanium oxide | | 5.0 |
| 13 | tocopherol | | 0.2 |
| 14 | alkylsilicone-branched silicone⁶⁾-treated talc | | 4.0 |
| 15 | alkylsilicone-branched silicone⁶⁾-treated sericite | | 2.0 |
| Total | | | 100.0 |
| total of component (a) | | | 4.0 |
| total of component (b) | | | 56.6 |
| total of component (c) | | | 8.0 |
| total of component (d) | | | 0.5 |
| total of component (e) | | | 1.5 |

| | | | |
|---|---|---|---|
| 1) KP-545 (product of Shin-Etsu Chemical Co., Ltd.); a solution containing (alkyl acrylate / dimethicone) copolymer in decamethylcyclopentasiloxane; (alkyl acrylate / dimethicone) copolymer : decamethylcyclopentasiloxane = 30:70 (wt/wt) 2) KSG-710 (product of Shin-Etsu Chemical Co., Ltd.); a composition containing a partially cross-linked polyglycerin-modified silicone swollen with dimethylpolysiloxane; partially cross-linked polyglycerin-modified silicone : dimethylpolysiloxane = 25:75 (wt/wt) 3) KSP-101 (product of Shin-Etsu Chemical Co., Ltd.); hybrid silicone composite powder 4) KF-56A (product of Shin-Etsu Chemical Co., Ltd.); diphenylsiloxyphenyl trimethicone 5) AEROSIL R972 (product of Nippon Aerosil Co., Ltd.); amorphous anhydrous silicic acid 6) KF-9909 (product of Shin-Etsu Chemical Co., Ltd.); alkylsilicone-branched silicone | | | |

### -Preparation method-

A foundation was prepared through the method comprising the following two steps:
step A: mixing a portion of the component 1, and the components 2, 3, 5 to 7 and 9 to 13 in Table 5, and evenly dispersing the mixed components by a roll mill; and
step B: evenly dispersing the rest of the component 1, the components 4, 8, 14 and 15 in Table 5 into the mixture obtained in the step A.

The foundation obtained in Example 3 in the form of a soufflé exhibited an excellent removability and a light spread, and was free of excessive oiliness and powdery touch, providing an excellent feeling in use. The foundation also exhibited an excellent water-resistance, water-repellency and perspiration resistance, as well as a favorable wearability, which makes a cosmetic not easily come off. Further, the foundation exhibited no change in the properties caused by temperature variation or aging. The excellent preservability of the cheek-color was proved.

### <Example 4> Eye-color

An eye-color was prepared according to the formulations shown in Table 6.

**[Table 6]**

| Component | | | % by mass |
|---|---|---|---|
| 1 | isododecane | | 30.5 |
| 2 | (alkyl acrylate / dimethicone) copolymer solution¹⁾ | (e) | 8.0 |
| | | | 12.0 |
| 3 | long chain alkyl-containing acrylic silicone resin²⁾ | | 2.0 |
| 4 | hybrid silicone composite powder³⁾ | (c) | 6.0 |
| 5 | trimethyl trimethicone⁴⁾ | (b) | 3.0 |
| 6 | vaseline | | 5.0 |
| 7 | alkyl-modified partially cross-linked polyether-modified silicone swollen composition⁵⁾ | (a) | 1.25 |
| | | | 3.75 |
| 8 | amorphous anhydrous silicic acid⁶⁾ | (d) | 1.0 |
| 9 | barium sulfate | | 5.0 |
| 10 | organic pigment | | 0.2 |
| 11 | alkylsilicone-branched silicone⁷⁾-treated iron oxide | | 1.0 |
| 12 | alkylsilicone-branched silicone⁷⁾-treated iron titanium | | 1.0 |
| 13 | alkylsilicone-branched silicone⁷⁾-treated titanated mica | | 20.0 |
| 14 | tocopherol | | 0.2 |
| 15 | perfume | | 0.1 |
| Total | | | 100.0 |
| total of component (a) | | | 1.25 |
| total of component (b) | | | 3.0 |
| total of component (c) | | | 6.0 |
| total of component (d) | | | 1.0 |
| total of component (e) | | | 8.0 |

| | | | |
|---|---|---|---|
| 1) KP-550 (product of Shin-Etsu Chemical Co., Ltd.); a solution containing (alkyl acrylate / dimethicone) copolymer in isododecane; (alkyl acrylate / dimethicone) copolymer : isododecane = 40:60 (wt/wt) 2) KP-561P (product of Shin-Etsu Chemical Co., Ltd.); long chain alkyl-containing acrylic silicone resin 3) KSP-441 (product of Shin-Etsu Chemical Co., Ltd.); hybrid silicone composite powder 4) TMF-1.5 (product of Shin-Etsu Chemical Co., Ltd.); trimethyl trimethicone 5) KSG-320 (product of Shin-Etsu Chemical Co., Ltd.); a composition containing an alkyl-modified partially cross-linked polyether-modified silicone swollen with isododecane; alkyl-modified partially cross-linked polyether-modified silicone: isododecane = 25:75 (wt/wt) 6) AEROSIL R972 (product of Nippon Aerosil Co., Ltd.); amorphous anhydrous silicic acid 7) KF-9909 (product of Shin-Etsu Chemical Co., Ltd.); alkylsilicone-branched silicone | | | |

### -Preparation method-

An eye-color was prepared through the method comprising the following two steps:
step A: mixing the components 1 to 8 in Table 6, and evenly dispersing the mixed components; and
step B: evenly dispersing the components 9 to 15 in Table 6 into the mixture obtained in the step A.

The eye-color obtained in Example 4 exhibited an excellent removability and a light spread, and was free of excessive oiliness and powdery touch, providing an excellent feeling in use. The eye-color also exhibited an excellent water-resistance, water-repellency and perspiration resistance, as well as a favorable wearability, which makes a cosmetic not easily come off. Further, the eye-color exhibited no change in the properties caused by temperature variation or aging. An excellent preservability of the eye-color was proved.

### [Industrial applicability]

The oil-based cosmetic preparation of the present invention is useful for oil-based base-makeup cosmetic preparations such as foundation and face-color, and for oil-based point-makeup cosmetic preparations such as eye-color, eye-liner, and cheek-color.

## Claims

1. An oil-based makeup cosmetic preparation comprising the following components (a), (b), (c) and (d) each in the following amount relative to the entire mass of the cosmetic preparation:
(a) a partially cross-linked polyether-modified silicone, a partially cross-linked polyglycerin-modified silicone, or a combination thereof, in an amount of from 1 to 7.5 % by mass;
(b) a low viscosity silicone oil with a viscosity of from 1 to 100 mm²/s at 25 °C in an amount of from 3 to 75 % by mass;
(c) a cross-linked silicone powder in an amount of from 0.1 to 18 % by mass; and
(d) an amorphous anhydrous silicic acid with an average particle size of from 0.001 to 0.1 µm in an amount of from 0.1 to 5 % by mass.

2. The oil-based makeup cosmetic preparation of claim 1, wherein the total amount of the components (a), (b), (c) and (d) is from 4.2 to 100 % by mass relative to the entire mass of the cosmetic preparation.

3. The oil-based makeup cosmetic preparation of claim 1 or 2, wherein the cosmetic preparation further comprises a silicone film-forming agent (e) to form a water-resistant film on the skin.

4. The oil-based makeup cosmetic preparation of any of claims 1 to 3, wherein the cross-linked silicone powder (c) has a rubber hardness of less than 80 as measured with a type-A durometer as provided by JIS K6253, and is selected from the group consisting of silicone rubber powders and a silicone resin-coated silicone rubber powders.

5. The oil-based makeup cosmetic preparation of any of claims 1 to 4, wherein the cosmetic preparation has a rheometer hardness of less than 50 as measured with Rheometer RT-2002D·D (product of RHEOTEC Co. Ltd.) at 25 °C.

## Patentansprüche

1. Kosmetisches Makeup-Präparat auf Ölbasis, umfassend die folgenden Komponenten (a), (b), (c) und (d) jeweils in den folgenden Mengen bezogen auf die Gesamtmasse des kosmetischen Präparats:
(a) ein teilweise vernetztes Polyethermodifiziertes Silicon, ein teilweise vernetztes Polyglycerin-modifiziertes Silicon oder eine Kombination davon in einer Menge von 1 bis 7,5 Masse-%;
(b) ein niederviskoses Siliconöl mit einer Viskosität von 1 bis 100 mm²/s bei 25 °C in einer Menge von 3 bis 75 Masse-%;
(c) ein vernetztes Siliconpulver in einer Menge von 0,1 bis 18 Masse-%; und
(d) eine amorphe wasserfreie Kieselsäure mit einer mittleren Partikelgröße von 0,001 bis 0,1 µm in einer Menge von 0,1 bis 5 Masse-%.

2. Kosmetisches Makeup-Präparat auf Ölbasis gemäß Anspruch 1, wobei die Gesamtmenge der Komponenten (a), (b), (c) und (d) von 4,2 bis 100 Masse-% bezogen auf die Gesamtmasse des kosmetischen Präparats beträgt.

3. Kosmetisches Makeup-Präparat auf Ölbasis gemäß Anspruch 1 oder 2, wobei das kosmetische Präparat ferner einen Silicon-Filmbildner (e) zum Bilden eines wasserbeständigen Films auf der Haut umfasst.

4. Kosmetisches Makeup-Präparat auf Ölbasis gemäß einem der Ansprüche 1 bis 3, wobei das vernetzte Siliconpulver (c) eine Kautschukhärte von weniger als 80 wie gemessen mit einem Typ-A-Durometer gemäß JIS K6253 aufweist und ausgewählt ist aus der Gruppe bestehend aus Siliconkautschukpulvern und Siliconharz-beschichteten Siliconkautschukpulvern.

5. Kosmetisches Makeup-Präparat auf Ölbasis gemäß einem der Ansprüche 1 bis 4, wobei das kosmetische Präparat eine Rheometer-Härte von weniger als 50 wie gemessen mit einem Rheometer RT-2002D-D (Produkt von RHEOTEC Co. Ltd.) bei 25 °C aufweist.

## Revendications

1. Préparation cosmétique de maquillage à base d'huile, comprenant les composants (a), (b), (c) et (d) suivants, chacun selon la quantité suivante par rapport à l'ensemble de la masse de la préparation cosmétique :
(a) une silicone à modification polyéther et partiellement réticulée, une silicone à modification polyglycérol et partiellement réticulée, ou une combinaison de celles-ci, selon une quantité allant de 1 à 7,5% en masse ;
(b) une huile de silicone de faible viscosité, ayant une viscosité allant de 1 à 100 mm²/s à 25°C, selon une quantité allant de 3 à 75% en masse ;
(c) une poudre de silicone réticulée, selon une quantité allant de 0,1 à 18% en masse ; et
(d) un acide silicique anhydre amorphe ayant une taille moyenne de particules allant de 0,001 à 0,1 µm, selon une quantité allant de 0,1 à 5% en masse.

2. Préparation cosmétique de maquillage à base d'huile selon la revendication 1, où la quantité totale des composants (a), (b), (c) et (d) va de 4,2 à 100% en masse par rapport à l'ensemble de la masse de la préparation cosmétique.

3. Préparation cosmétique de maquillage à base d'huile selon la revendication 1 ou 2, où la préparation cosmétique comprend en outre un agent filmogène de silicone (e) afin de former un film résistant à l'eau sur la peau.

4. Préparation cosmétique de maquillage à base d'huile selon l'une quelconque des revendications 1 à 3, où la poudre de silicone réticulée (c) possède une dureté de caoutchouc inférieure à 80, telle que mesurée à l'aide d'un duromètre de type A tel que fourni par JIS K6253, et est choisie dans le groupe constitué par les poudres de caoutchouc de silicone et les poudres de caoutchouc de silicone revêtu d'une résine de silicone.

5. Préparation cosmétique de maquillage à base d'huile selon l'une quelconque des revendications 1 à 4, où la préparation cosmétique possède une dureté par rhéomètre inférieure à 50, telle que mesurée à l'aide d'un rhéomètre RT-2002D·D (produit de RHEOTEC Co. Ltd) à 25°C.
